# EUROPEAN PATENT SPECIFICATION

(11) **EP 1 862 228 B1**
(45) Date of publication and mention of the grant of the patent: **28.03.2012**
(21) Application number: 07107297.9
(22) Date of filing: 01.05.2007
(51) Int. Cl.: B07C 5/342, G01N 21/90, G01B 11/06

(54) **Out of round detector**
Radialschlagdetektor
Détecteur d'excentricité

(30) Priority: 02.06.2006 US 445893
(43) Date of publication of application: 05.12.2007
(73) Proprietor: Emhart Glass S.A., 6330 Cham (CH)
(72) Inventor: Weber, Gary C., Horseheads, NY 14845 (US); Furnas, William J., Elmira, NY 14901 (US); Diehr, Richard D., Horseheads, NY 14845 (US)
(74) Representative: Warren, Anthony Robert

(56) References cited:
- EP-A- 0 248 552
- EP-A- 0 320 139
- EP-A- 1 288 613
- GB-A- 2 182 436
- US-A1- 2006 006 352
- US-A1- 2006 098 191
- US-B1- 6 975 410

## Description

The present invention relates to inspection machines for verifying that a formed container, such as a bottle, has a desired cylindrical shape.

Cylindrical glass bottles formed in an I. S. machine must satisfy any number of functional and appearance qualifications, One of these is that the bottle should be cylindrical and when it is "out of round" to a selected degree, the bottle is rejected. Patented technologies can be seen in U.S. Patents numbered 4,368,641, and 5,414,939.

In addition, EP-A-1,288,613 discloses apparatus for measuring the sidewall thickness of transparent containers which includes a laser for directing a line-shaped laser beam onto the outer surface of the sidewall of the rotating container at an oblique angle such that a portion of the beam is reflected from the outer sidewall surface, and a portion is refracted and reflected from the inner sidewall surface. The reflected beam portions from the inner and outer sidewall surfaces are directed through a lens system onto a linear array light sensor. An information processor connected to the light sensor measures the container wall thickness as a function of the separation at the light sensor between the beam portions.

US-2006/006352 A1 discloses apparatus for inspecting ribbed containers which also includes a laser for directing a line-shaped laser beam onto the outer surface of a rotating container at an oblique angle. The line shape of the beam has a long dimension, parallel to the container axis sufficient to illuminate at least one rib peak and one valley between ribs. The beam is reflected by the outer surface of the container and is direct through a lens system to a light sensor which is scanned by an information processor. An image is formed on the sensor comprising image elements representing the reflections from a rib peak and a rib valley on the outer surface of the container sidewall and from the inner surface of the sidewall. The spacing between the image elements relates to the distance between the container sidewall and the inspection apparatus whilst relative movement of the image elements is representative of out-of-round conditions.

The present invention is directed to an inspection machine for determining whether a container when located at an inspection station is "out-of-round", generally of the type disclosed in US-2006/006352 A1 and as recited in the preamble of claim 1.

It is an object of the present invention to provide an out of round detector which is very simple to implement.

According to the present invention, there is provided an inspection machine as defined in the characterizing clause of claim 1.

Reference will now be made to the accompanying drawings which illustrate a presently preferred embodiment incorporating the principles of the invention, and in which:-
Figure 1 is a schematic representation of an out of round inspection machine made in accordance with the teachings of the present invention;
Figure 2 is a schematic showing of the out of round inspection in accordance with the teachings of the present invention; and
Figure 3 is a schematic showing of the out of round inspection with the camera viewing line and the laser beam defining a vertical angle.

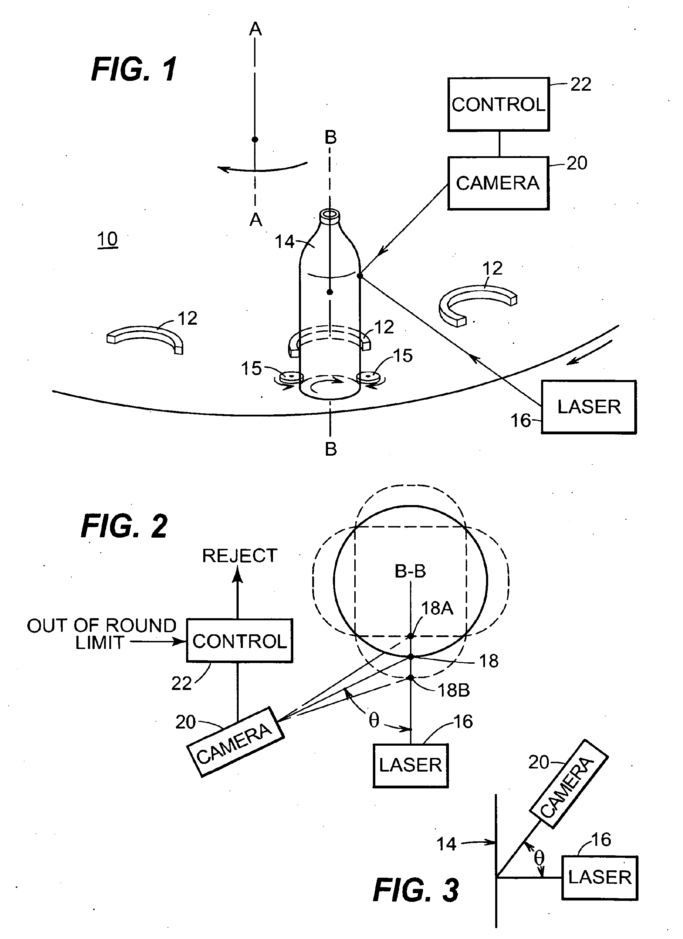

Figure 1 discloses an inspection machine having a support table 10 rotatable about a vertical axis A-A. The table is indexed from position to position and inspections can take place at any position (pockets 12 represent the discrete locations of bottles 14 on the table) and the illustrated bottle is shown located at the "out of round" inspection position. The bottle will be rotated about its axis B-B, by bottle rotators 15, while the bottle is located at the out of round inspection position. These rotors are a part of an overall rotator means or mechanism made up of the rotors and their drive.

At the inspection position a laser 16 directs a beam at the wall of the bottle. As can be seen from Figure 2, this laser beam is directed at the axis B-B of the bottle. The laser beam creates a small spot 18 of diffused light at the bottle surface and a Camera 20 such as a CCD camera is aimed at an angle Ø to the laser beam to see that spot. As the bottle rotates about its axis, the location of this spot on the CCD image field of the camera will vary as the spot moves between location 18A where the rotating bottle will have a minimum diameter and location 18B where the rotating bottle will have a maximum diameter. The displacement distance of the spot as the bottle rotates, which is computed by a Control 22, indicates how much the bottle is out of round, and an Out Of Round Limit defining the maximum displacement can be input by the operator so that a reject signal will be generated for a bottle that is beyond the acceptable out of round limit. While the illustrated angle Ø is shown in a horizontal plane, it can also be in a vertical or other plane as shown in Figure 3 where the surface of the glass bottle 14 being inspected is vertical.

## Claims

1. An inspection machine for determining whether a container (14) when located at an inspection station is "out of round", comprising
a laser (16), spaced from the container to be inspected, for directing a laser beam towards the container to be inspected to impinge on the surface of the container to be inspected,
rotator means (15) for rotating the container to be inspected about its axis,
a camera (20) for viewing, at a defined angle (Ø) to the laser beam, the location where the laser beam impinges on the surface of the container, and
a control (22) for determining the displacement of said location as the container to be inspected is rotated 360 degrees about its axis and for issuing a reject signal in the event the displacement exceeds a set limit,
**characterized in that**:
the laser (16) is positioned to direct the laser beam perpendicularly towards the axis (B-B) of the container (14) to be inspected whereby the laser beam defines said location of impingement as a spot of energy (18) on the surface of the container.

2. An inspection machine according to claim 1, wherein said defined angle (Ø) lies within a horizontal plane.

3. An inspection machine according to claim 1, wherein said defined angle (Ø) lies within a vertical plane.

4. An inspection machine according to claim 1, 2 or 3, wherein the camera (20) is a CCD camera.

5. An inspection machine according to any of claims 1 to 4, wherein the container (14) to be inspected is a cylindrical glass bottle.

## Patentansprüche

1. Prüfmaschine zum bestimmen, ob ein an einer Prüfstation befindlicher Behälter (14) "unrund" ist, umfassend
einen Laser (16) der vom zu prüfenden Behälter beabstandet ist und einen Laserstrahl auf den zu prüfenden Behälter richtet, der auf die Oberfläche des zu prüfenden Behälters auftrifft,
eine Dreheinrichtung (15) zum Drehen des zu prüfenden Behälters um seine Achse,
eine Kamera (20) zur Betrachtung des Ortes, wo der Laserstrahl auf die Oberfläche des Behälters auftrifft, in einem zum Laserstrahl definierten Winkel (Ø) und
eine Steuerung (22) zum Bestimmen der Verschiebung des Ortes, wenn der zu prüfende Behälter um 360 °C um seine Achse gedreht wird, und zum Abgeben eines Zurückweisungssignals, falls die Verschiebung eine vorbestimmte Grenze überschreitet,
**dadurch gekennzeichnet, dass**
der Laser (16) so angeordnet ist, dass der Laserstrahl senkrecht zur Achse (B-B) des zu prüfenden Behälters (14) gerichtet wird, wobei der Laserstrahl den Ort des Auftreffens als Energiepunkt (18) auf der Oberfläche des Behälters definiert.

2. Prüfmaschine nach Anspruch 1, wobei der definierte Winkel (Ø) in einer horizontalen Ebene liegt.

3. Prüfmaschine nach Anspruch 1, wobei der definierte Winkel (Ø) in einer vertikalen Ebene liegt.

4. Prüfmaschine nach Anspruch 1, 2 oder 3, wobei die Kamera (20) eine CCD Kamera ist.

5. Prüfmaschine nach einem der Ansprüche 1-4, wobei der zu prüfende Behälter (14) eine zylindrische Glasflasche ist.

## Revendications

1. Machine d'inspection pour déterminer si un récipient (14), lorsqu'il est situé au niveau d'un poste d'inspection, « présente une excentricité », comprenant :
un laser (16), espacé du récipient à inspecter, pour diriger un faisceau laser vers le récipient à inspecter pour qu'il frappe la surface du récipient à inspecter,
des moyens de rotation (15) pour faire tourner le récipient à inspecter autour de son axe,
une caméra (20) pour observer, selon un angle défini (Ø) par rapport au faisceau laser, l'emplacement où le faisceau laser frappe la surface du récipient, et
une commande (22) pour déterminer le déplacement dudit emplacement alors que le récipient à inspecter est tourné de 360 degrés autour de son axe et pour émettre un signal de rejet dans le cas où le déplacement dépasse une limite fixée,
**caractérisée en ce que** :
le laser (16) est positionné de manière à diriger le faisceau laser perpendiculairement à l'axe (B-B) du récipient (14) à inspecter, moyennant quoi le faisceau laser définit ledit emplacement d'impact en tant que point d'énergie (18) sur la surface du récipient.

2. Machine d'inspection selon la revendication 1, dans laquelle ledit angle défini (Ø) se trouve dans un plan horizontal.

3. Machine d'inspection selon la revendication 1, dans laquelle ledit angle défini (Ø) se trouve dans un plan vertical.

4. Machine d'inspection selon la revendication 1, 2 ou 3, dans laquelle la caméra (20) est une caméra CCD.

5. Machine d'inspection selon l'une quelconque des revendications 1 à 4, dans laquelle le récipient (14) à inspecter est une bouteille en verre cylindrique.
